# EUROPEAN PATENT SPECIFICATION

(11) **EP 3 795 138 B1**
(45) Date of publication and mention of the grant of the patent: **15.05.2024**
(21) Application number: 20205915.0
(22) Date of filing: 13.07.2018
(51) Int. Cl.: A61K 8/87, A61K 8/81, A61K 8/37, A61Q 17/04, A61K 8/34, A61K 8/35, A61K 8/40, A61K 8/49, A61K 8/895

(54) **LIGHT AESTHETIC SUNSCREEN COMPOSITIONS**
LEICHTE ÄSTHETISCHE SONNENSCHUTZZUSAMMENSETZUNGEN
COMPOSITIONS D'ÉCRAN SOLAIRE À ESTHÉTIQUE LUMINEUSE

(30) Priority: 13.07.2017 US 201715648494; 12.07.2018 US 201816033528
(43) Date of publication of application: 24.03.2021
(62) Divisional of application: 18749942.1
(73) Proprietor: Johnson & Johnson Consumer Inc., Skillman, NJ 08558 (US)
(72) Inventor: ZANATTA, Cinthia, Skillman, NJ New Jersey 08558 (US); MARTIN, Jeffrey Daniel, Skillman, NJ New Jersey 08558 (US); CONSUL DE MORAES, Alice Aparecida, 12243- 260 Sao Jose Dos Campos/SP (BR)
(74) Representative: Carpmaels & Ransford LLP

(56) References cited:
- US-A1- 2011 064 681
- US-A1- 2017 128 357

## Description

### Background of the Invention

It is well known that prolonged exposure to ultraviolet radiation, such as from the sun, can lead to the formation of light dermatoses and erythemas, and increase the risk of skin cancers, such as melanoma. Exposure to UV radiation also accelerates skin aging, such as loss of skin elasticity and wrinkling.

For these reasons, sunscreen compositions are commonly used to provide protection from the sun, and a variety of sunscreen compositions are commercially available. Unfortunately, most have a heavy, oily aesthetic because high levels of oil are required to carry the load of UV filters needed to achieve an acceptable SPF. The heavy, oily feel can be unpleasant and discourage users from applying enough product to provide good protection from the sun. It would therefore be desirable to provide a sunscreen composition having both an excellent SPF and a light, watery, pleasant aesthetic.

It has now been discovered that a sunscreen composition having a watery break on topical application may be prepared using a combination of a hydrophobically modified polyurethane, viscosity increasing polymer selected from the group consisting of taurate copolymers and acrylate crosspolymers, and glyceryl stearate.

### Summary of the Invention

The present invention provides a sunscreen composition comprising: (a) at least about 10 weight percent of one or more UV filters; (b) about 0.55 to about 0.75 weight percent of a hydrophobically modified polyurethane; (c) about 0.1 to about 0.6 weight percent of a viscosity increasing polymer selected from the group consisting of taurate copolymers and acrylate crosspolymers; and (d) at least about 0.1 weight percent of glyceryl stearate; wherein said viscosity increasing polymer is a taurate copolymer.

The invention further provides a sunscreen composition comprising at least about 10 weight percent of one or more UV filters and having a linear complex viscosity of 1500 to 5000 cP at 100 rad/s.

The invention also provides a sunscreen composition comprising:
(a) at least about 10 weight percent of a combination of UV filters comprising avobenzone, octocrylene, homosalate, and octisalate;
(b) about 0.55 to about 0.75 weight percent of a hydrophobically modified polyurethane;
(c) about 0.4 to about 0.6 weight percent of a taurate copolymer; and
(d) at least about 0.1 weight percent of glyceryl stearate;
(e) silica;
(f) a humectant selected from the group consisting of glycerin, pentylene glycol, and mixtures thereof; and
(g) ethanol.

### Detailed Description of the Invention

Unless defined otherwise, all technical and scientific terms used herein have the same meaning as commonly understood by one of ordinary skill in the art to which the invention belongs.

As used herein, "topically applying" means directly laying on or spreading on outer skin or the scalp, e.g., by use of the hands or an applicator such as a wipe, roller, or spray.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically effective amount" means an amount of a physiologically active compound or composition sufficient for treating one or more conditions, but low enough to avoid serious side effects. The cosmetically effective amount of the compound or composition will vary with the condition being treated, the age and physical condition of the end user, the severity of the condition being treated/prevented, the duration of the treatment, the nature of other treatments, the specific compound or product/composition employed, the cosmetically-acceptable carrier utilized, and like factors.

As used herein, "cosmetically acceptable" means that the ingredients the term describes are suitable for use in contact with tissues (e.g., the skin) without undue toxicity, incompatibility, instability, irritation, allergic response, or the like.

As used herein, a "cosmetically acceptable active agent" is a compound (synthetic or natural) that has a cosmetic or therapeutic effect on the skin.

As used herein, "treatment or treating" refers to mitigating, reducing, preventing, improving, or eliminating the presence or signs of a condition or disorder.

As used herein, "phase-stable" means the water and oil phases of an emulsion do not appreciably separate at room temperature for at least two weeks.

Unless otherwise indicated, a percentage or concentration refers to a percentage or concentration by weight (i.e., % (W/W). Unless stated otherwise, all ranges are inclusive of the endpoints, e.g., "from 4 to 9" includes the endpoints 4 and 9.

### UV FILTER

The sunscreen composition comprises at least about 10 weight percent of one or more UV filters. In one embodiment, the composition comprises at least about 15 to about 35 weight percent of one or more UV filters.

As used herein, "UV filter" is a material or compound, polymeric or non-polymeric, that absorbs, scatters or reflects radiation in some portion of the ultraviolet spectrum (290nm-400nm), such as one having an extinction coefficient of at least about 1000 mol-1 cm-1 for at least one wavelength within the ultraviolet spectrum. SPF values disclosed and claimed herein are determined using the in-vitro method described herein below.

The UV filters may be "organic" UV filters. Organic UV filters, often referred to as "monomeric, organic UV-absorbers" are generally aromatic compounds conjugated with a carbonyl moiety substituted in the ortho- or para- position of the aromatic ring.

Traditional organic UV filters are aromatic, small molecules, with molecular weight values <900 g/mol. Examples of organic non-polymeric UV filters include, but are not limited to: methoxycinnamate derivatives such as octyl methoxycinnamate and isoamyl methoxycinnamate; camphor derivatives such as 4-methyl benzylidene camphor, camphor benzalkonium methosulfate, ecamsule (Mexoryl^{®} SX); terephthalylidene dicamphor sulfonic acid,and terephthalylidene dicamphor sulfonic acid; salicylate derivatives such as octyl salicylate, trolamine salicylate, ethylhexyl salicylate and homosalate; sulfonic acid derivatives such as phenylbenzimidazole sulfonic acid; benzone derivatives such as dioxybenzone, sulisobenzone, and oxybenzone; benzoic acid derivatives such as aminobenzoic acid and octyldimethyl para-amino benzoic acid; octocrylene and other β,β-diphenylacrylates; dioctyl butamido triazone; octyl triazone; avobenzone (butyl methoxydibenzoylmethane); drometrizole trisiloxane; menthyl anthranilate; triazone derivatives such as ethylhexyl triazone (Uvinul^{®} T150); diethylhexyl butamido triazone (UVASorb^{®} HEB); bis-ethylhexyloxyphenol methoxyphenyl triazine (Tinosorb^{®} S), benzoate derivatives such as diethylamino hydroxybenzoyl hexyl benzoate (Uvinul^{®} A Plus), benzotriazole derivatives such as drometrizole trisiloxane (Mexoryl^{®} XL), methylene bis-benzotriazolyl tetramethylbutylphenol; tris-biphenyl triazine; (2-{4-[2-(4-Diethylamino-2-hydroxy-benzoyl)-benzoyl]-piperazine-1-carbonyl}-phenyl)-(4-diethylamino-2-hydroxyphenyl)-methanone; merocyanine derivatives; bis(butylbenzoate) diaminotriazine aminopropylsiloxane; and bis-ethylhexyloxyphenol methoxyphenyl triazine, encapsulated in a polymer matrix.

In certain embodiments of the invention, the sunscreen composition comprises one or more UV filters selected from Table 1.

**Table 1**

| **UV Filter** | **Other names** | **Coverage** |
|---|---|---|
| | | |
| Benzophenone-3 | **Oxybenzone** or 2-hydroxy-4-methoxybenzophenone | UVA/B |
| Benzophenone-4 | **Sulizobenzone** or 2-hydroxy-4-methoxybenzophenone-5-sulfonic acid and its trihydrate | UVA/B |
| Benzophenone-5 | 2-hydroxy-4-methoxybenzophenone-5-sulfonic acid (benzophenone-5) and its sodium salt | UVA/B |
| | Sulizobenzone sodium | |
| | Sodium hydroxymethoxybenzophenone sulfonate | |
| Benzophenone-8 | Dioxybenzone or 2,2'-dihydroxy-4-methoxybenzophenone | UVA/B |
| | dioxybenzone | |
| | (2-hydroxy-4-methoxyphenyl)(2-hydroxyphenyl)methanone | |
| | methanone, (2-hydroxy-4-methoxyphenyl)(2-hydroxyphenyl) | |
| 3-benzylidene camphor | 3-benzylidene camphor | UVB |
| | | |
| Bis ethylhexyloxyphenol methoxyphenyl triazine | **Tinosorb S** or (1,3,5)-triazine-2,4-bis{[4-(2-ethyl-hexyloxy)-2-hydroxy]-phenyl}-6-(4-methoxyphenyl) or anisotriazine | UVA/B |
| Butylmethoxy dibenzoyl methane | **Avobenzone** or 1-(4-tert-butylphenyl)-3-(4-methoxyphenyl) propane-1,3-dione | UVA |
| Camphor benzalkonium Methosulfate | **Mexoryl SO** or N,N,N-trimethyl-4-(2-oxoborn-3-ylidene-methyl) anilinium methyl sulphate | UVB |
| Diethylamino hydroxybenzoyl | **Uvinul A plus** or | UVA |
| hexyl benzoate | benzoic acid, 2-[-4-(diethylamino)-2-hydroxybenzoyl]-, hexylester | |
| Diethylhexyl butamido triazone | **UVASorb HEB** or | UVB |
| | benzoic acid, 4,4-((6-((4-(((1,1-dimethylethyl) amino) carbonyl) phenyl) amino) 1,3,5-triazine-2,4-diyl) diimino) bis - (2-) ester) | |
| | or dioctyl butamido triazone | |
| Disodium phenyl dibenzimidazole | **Neo Heliopan AP** or | UVA |
| Tetrasulfonate | monosodium salt of 2-2'-bis(1,4-phenylene)1H-benzimidazole-4,6-disulphonic acid) or | |
| | bisimidazylate | |
| | | |
| Drometrizole trisiloxane | **Mexoryl XL** or | UVA/B |
| | phenol,2-(2H-benzotriazol-2-yl)-4-methyl-6-(2-methyl-3-(1,3,3,3-tetramethyl-I-(trimethylsilyl)oxy)-disiloxanyl)propyl) | |
| Ethoxyethyl methoxycinnamate | Cinoxate | UVB |
| Ethylhexyl dimethylamino | Padimate O | UVB |
| | Octyl dimethyl **PABA** | |
| Benzoate | Ethylhexyl dimethyl PABA | |
| Ethylhexyl methoxycinnamate | **OMC** or octinoxate | UVB |
| | Octyl methoxycinnamate | |
| Ethylhexyl salicylate | **Octisalate** | UVB |
| | 2-ethylhexyl salicylate | |
| | Octyl salicylate | |
| | | |
| Ethylhexyl triazone | **Uvinul T150** | UVB |
| | 2,4,6-trianilino-(p-carbo-2'-ethylhexyl-l'oxy)-1,3,5-triazine | |
| | Octyl triazone | |
| Homosalate | 3,3,5-trimethylcyclohexyl 2-hydroxybenzoate | UVB |
| | Salicilato de homomentila | |
| Isoamyl p-methoxycinnamate | Amiloxate | UVB |
| | Isopentyl-4-methoxycinnamate | |
| Methyl anthranilate | Meradimate | UVA |
| 4-methylbenzylidene camphor | Enzacamene | UVB |
| | 3-(4'-methylbenxylidene)d-1 camphor 4 MBC | |
| | | |
| Methylene bis-benzotriazolyl | **Tinosorb M** | UVA/B |
| tetramethylbutylphenol | 2,2'-methylene-bis-6-(2H-benzotriazol-2yl)-4-(tetramethyl-butyl)-1,1,3,3-phenol | |
| Octocrylene | 2-cyano-3,3-diphenyl acrylic acid, 2-ethylhexyl ester | UVB |
| Para aminobenzoic acid | **PABA** | UVB |
| | 4-aminobenzoic acid | |
| PEG-25 **PABA** | Ethoxylated ethyl-4-aminobenzoate | UVB |
| Phenyl benzimidazole | **Neo Heliopan Hydro** - Ensulizole | UVB |
| sulfonic acid | 2-phenylbenzimidazole-5-sulphonic acid and its potassium, sodium, and triethanolamine salts | |
| | Potassium, Sodium, and TEA Phenylbenzimidazole sulfonate | |
| Polyacrylamido methyl benzylidene | **Mexoryl SW** | UVB |
| Camphor | Polymer of N-[(2 and 4)-[(2-oxoborn-3-ylidene)methyl]benzyl]acrylamide | |
| | | |
| Polysilicone-15 | **Parsol SLX** | UVB |
| | Diethylbenzylidene malonate Dimethicone | |
| | Diethylmalonylbenzylidene Oxypropene dimethicone | |
| | Dimethicodiethylbenzalmalonate | |
| Triethanolamine salicylate | **Neo Heliopan TES** | UVB |
| | Trolamine salicylate | |
| Terephtalydene dicamphor | **Mexoryl SX** | UVA |
| sulfonic acid | | |
| Benzylidene camphor sulfonic acid | Alpha-(2-oxoborn-3-ylidene)-toluene-4-sulfonic acid and its salts | |
| Titanium dioxide | | UVA/B |
| Zinc oxide | | UVA/B |

The organic UV filter may alternatively be a polymer made of organic chromophores attached to a polysiloxane chain having for example an average molecular weight of >6000 daltons. Examples of such polysiloxanes compounds include, without limitation to, Parsol^{®} SLX and polysilicone-15. These polysiloxanes absorb in the UVB (λmax = 312 nm) part of the spectrum and are typically combined with UVA filters to achieve broad-spectrum protection.

Alternatively, the UV filter may be a UV blocker. UV blockers reflect, absorb or scatter the UV radiation and if present in sunscreen compositions, can reflect all the ultraviolet, visible and infrared rays that enhance sun protection. UV blockers are typically inorganic metallic oxides, including titanium dioxide, zinc oxide, and certain other transition metal oxides. Such UV blockers are typically solid particles in a micronized or nanonized form having a diameter from about 0.01 micron to about 10 microns.

In one embodiment of the present invention, the sunscreen composition comprises avobenzone.

In another embodiment, the sunscreen composition comprises a combination of UV filters comprising avobenzone, octocrylene, and homosalate.

In another embodiment, the sunscreen composition comprises a combination of UV filters comprising avobenzone, octocrylene, homosalate, and octisalate.

In a further embodiment, the sunscreen composition comprises a combination of UV filters comprising avobenzone, octocrylene, homosalate, and a triazone derivative. For example, the triazone derivative may be bis-ethylhexyloxyphenol methoxyphenyl triazine.

The SPF of the sunscreen composition may be determined using the USA FDA Final Rule test method, No. FDA 1978-N-0018, known to those skilled in the sunscreen art. In one embodiment, the sunscreen composition has an SPF of at least about 15. In another embodiment, the sunscreen composition has an SPF of at least about 25.

The total amount of UV filters in the sunscreen composition is at least about 10 weight percent. In one embodiment, the total amount of UV filters in the sunscreen composition is at least about 15 weight percent.

### HYDROPHOBICALLY MODIFIED POLYURETHANE

The sunscreen composition also contains about 0.55 to about 0.75 + weight percent of a hydrophobically modified polyurethane.

In one embodiment, the hydrophobically modified polyurethane is polyurethane-62. Polyurethane-62 is commercially available as AVALURE^{™} Flex-6 Polymer, a solution of polyurethane-62 in trideceth-6, from Lubrizol Advanced Materials, Inc., Cleveland, OH.

### VISCOSITY INCREASING POLYMER

The composition also contains one or more viscosity increasing polymers. In one embodiment, the viscosity increasing polymer is selected from the group consisting of taurate copolymers.

In one embodiment, the viscosity increasing polymer comprises a taurate copolymer. The composition may contain about 0.4 to about 0.6 weight percent of a taurate copolymer.

In one embodiment, the taurate copolymer is an acryloyldimethyltaurate/vinylpyrrolidine crosspolymer.

In one embodiment, the taurate copolymer is a sodium acryloyldimethyltaurate/vinylpyrrolidine crosspolymer. Sodium acryloyldimethyltaurate/vinylpyrrolidine crosspolymer is commercially available for example as ARISTOFLEX^{®} AVS from Clariant.

### GLYCERYL STEARATE

The composition further comprises at least about 0.1 weight percent of glyceryl stearate. In one embodiment, the composition comprises at least about 0.3 weight percent of glyceryl stearate.

Glyceryl Stearate is a known emulsifier. It is commercially available, for example, as Cutina^{®} GMS V from BASF.

### TOPICAL COMPOSITION

The sunscreen composition can be used by topically applying to a mammal, e.g., by the direct laying on, wiping or spreading of the composition on the skin, hair, or nails of a mammal, particularly a human.

The sunscreen composition is in the form of an oil-in-water emulsion containing a continuous water phase and a discontinuous oil phase dispersed within the continuous water phase.

The sunscreen composition may be prepared using mixing and blending methodology well known in the sunscreen and cosmetic art. In one embodiment, the sunscreen composition is produced by preparing an oil phase by mixing the UV filters with optional oil soluble or oil-miscible ingredients; and preparing a water phase, by mixing water and optional water-soluble or water-miscible ingredients. The oil phase and the water phase may then be mixed in a manner sufficient to disperse the oil phase substantially homogeneously in the water phase such that the water phase is continuous and the oil phase discontinuous.

In certain embodiments, the oil-in water emulsion is phase stable.

In certain embodiments, the UV filter is dissolved, as opposed to being dispersed or suspended, within the oil phase. Suitable solvents for the UV filters include dicaprylyl carbonate available as CETIOL CC from Cognis Corporation of Ambler, Pennsylvania.

The oil phase may be such that it is present in discrete droplets or units having an average diameter of about one micron to about 1000 microns, such as from about 1 micron to about 100 microns.

The percentage by weight of water phase included in the compositions may range from about 45% to about 90%, such as from about 55% to about 80%, such as from about 60% to about 80%. The percentage by weight of water in the water phase may be about 90% or more, such as about 95% or more.

In certain embodiments, the percentage by weight of oil phase in the composition is from about 10% to about 55%, such as from about 20% to about 45%, such as from about 20% to about 40%.

In certain embodiment, the oil phase consists essentially of the UV filter. In certain embodiments, the oil phase contains about 60% or more by weight of the UV filter, such as greater than 75% by weight of the UV filter, such as greater than 90% by weight of the UV filter, such as greater than 97% by weight of the UV filter, such as greater than 99% by weight of the UV filter.

The composition may be combined with a "cosmetically-acceptable topical carrier," i.e., a carrier for topical use that capable of containing the other ingredients dispersed or dissolved therein, and possessing acceptable properties rendering it safe to use topically.

The composition may optionally comprise a wide variety of additional oil-soluble materials and/or water-soluble materials conventionally used in compositions for use on skin, at their art-established levels. For example, surfactants, pearlescent or opacifying agents, thickeners, emollients, conditioners, humectants, chelating agents, exfoliants, and additives that enhance the appearance, feel, or fragrance of the cleansing composition, such as colorants, fragrances, preservatives, pH adjusting agents, and the like, can be included.

Water soluble or water dispersible polymers may be added to the compositions. The water dispersible polymers are comprised of a water-insoluble polymer that is typically micronized and dispersed into a water carrier, possibly with the use of a surface active dispersing aid. The water dispersible polymers are capable of forming a film and improving water resistance of the compositions. Examples of water soluble polymers include Polyaldo^{®} 10-1-L (Polyglyceryl-10 Laurate), available from Lonza. Examples of water dispersible polymers include water dispersible polyurethanes, such as Baycusan^{®} C1000 (Polyurethane-34), available from Bayer, Dow Corning^{®} 2501 (Bis-PEG-18 Methyl Ether Dimethyl Silane), available from Dow Corning, Eastman AQTM 38S (Polyester-5), available from Eastman Chemical, and Intelimer^{®} 8600 ( C8-22 Alkyl Acrylates/Methacrylic Acid Crosspolymer) available from Air Products.

The sunscreen composition may also comprise a film former. Film formers are typically hydrophobic materials that impart film forming and/or waterproofing characteristics. One such agent is polyethylene, which is available from New Phase Technologies as Performalene^{®} 400, a polyethylene having a molecular weight of 400. Another suitable film former is polyethylene 2000 (molecular weight of 2000), which is available from New Phase Technologies as Performalene^{®}. Another suitable film former is synthetic wax, also available from New Phase Technologies as Performa^{®} V-825. Other typical film-formers include acrylates/acrylamide copolymer, acrylates copolymer, acrylates/C12-C22 alkylmethacrylate copolymer, polyethylene, waxes, VP/dimethiconylacrylate/polycarbamylpolyglycol ester, butylated PVP, PVP/hexadecene copolymer, octadecene/MA copolymer, PVP/eicosene copolymer, tricontanyl PVP, Brassica Campestris/Aleuritis Fordi Oil copolymer, decamethyl cyclopentasiloxane (and) trimethylsiloxysilicate, Dimethicone; Acrylates/Dimethicone Copolymer and mixtures thereof. In some cases, the film former is acrylates/C12-C22 alkylmethacrylate copolymer sold under the tradename Allianz^{™} OPT by Ashland.

Suitable emollients include mineral oils, petrolatum, vegetable oils (e.g. triglycerides such as caprylic/capric triglyceride), waxes and other mixtures of fatty esters, including but not limited to esters of glycerol (e.g, isopropyl palmitate, isopropyl myristate, diisopropyl adipate, dicaprylyl carbonate), and silicone oils such as dimethicone. In certain embodiments, mixtures of triglycerides (e.g. caprylic/capric triclycerides) and esters of glycols (e.g. isopropyl myristate) may be used to solubilize the UV filters. In other embodiments of the invention, the compositions are essentially free of, or free of, emollients.

Examples of suitable solvents include propylene glycol, polyethylene glycol, polypropylene glycol, glycerol, 1,2,4-butanetriol, sorbitol esters, 1,2,6-hexanetriol, ethanol, and mixtures thereof. In one embodiment, the sunscreen composition comprises ethanol.

In certain embodiments, the composition includes a pigment suitable for providing color or hiding power. The pigment may be one suitable for use in a color cosmetic product, including compositions for application to the hair, nails and/or skin, especially the face. Color cosmetic compositions include, but are not limited to, foundations, concealers, primers, blush, mascara, eyeshadow, eyeliner, lipstick, nail polish and tinted moisturizers. The pigment suitable for providing color or hiding power may be composed of iron oxides, including red and yellow iron oxides, titanium dioxide, ultramarine and chromium or chromium hydroxide colors, and mixtures thereof. The pigment may be a lake pigment, e.g. an organic dye such as azo, indigoid, triphenylmethane, anthraquinone, and xanthine dyes that are designated as D&C and FD&C blues, browns, greens, oranges, reds, yellows, etc., precipitated onto inert binders such as insoluble salts. Examples of lake pigments include Red #6, Red #7, Yellow #5, Violet #2 and Blue #1. The pigment may be an interference pigment. Examples of interference pigments include those containing mica substrates, bismuth oxycloride substrates, and silica substrates, for instance mica/bismuth oxychloride/iron oxide pigments commercially available as CHROMALITE pigments (BASF), titanium dioxide and/or iron oxides coated onto mica such as commercially available FLAMENCO pigments (BASF), mica/titanium dioxide/iron oxide pigments including commercially available KTZ pigments (Kobo products), CELLINI pearl pigments (BASF), and borosilicate-containing pigments such as REFLECKS pigments (BASF).

In certain embodiments, the sunscreen composition may include one or more compounds suitable for enhancing the photostability of the UV filters of other ingredients in the composition. Photostabilizers include, for example, diesters or polyesters of a naphthalene dicarboxylic acid.

In one embodiment, the sunscreen composition comprises a humectant. Examples of humectants include, without limitation to, glycerin, pentylene glycol, butylene glycol. In another embodiment, the sunscreen composition comprises at least about 3 weight percent glycerin. The sunscreen composition may comprise for example at least about 2 weight percent, or at least about 3.5 weight percent glycerin. The sunscreen composition may comprise at least about 5 weight percent glycerin.

In one embodiment, the sunscreen composition comprises a humectant selected from the group consisting of glycerin, pentylene glycol, and mixtures thereof.

In another embodiment, the sunscreen composition has a pH of about 5 to about 6. The sunscreen composition may have a pH of about 5.5.

In a further embodiment, the sunscreen composition comprises hyaluronic acid. In another embodiment, the sunscreen composition comprises at least 0.01 weight percent hyaluronic acid.

The composition may comprise one or more other cosmetically acceptable active agents include for example anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, and agents for skin conditioning.

The amount of other cosmetically active agent in may range from about 0.001% to about 20% by weight of the composition, e.g., about 0.005% to about 10% by weight of the composition, such as about 0.01% to about 5% by weight of the composition.

The cosmetically acceptable active agent may be selected for instance from, benzoyl peroxide, D-panthenol carotenoids, ceramides, polyunsaturated fatty acids, essential fatty acids, enzymes such as laccase, enzyme inhibitors, minerals, hormones such as estrogens, steroids such as hydrocortisone, 2-dimethylaminoethanol, copper salts such as copper chloride, peptides like argireline, syn-ake and those containing copper, coenzyme Q10, amino acids such as proline, vitamins, lactobionic acid, acetyl-coenzyme A, niacin, riboflavin, thiamin, ribose, electron transporters such as NADH and FADH2, natural extracts such as from aloe vera, feverfew, oatmeal, dill, blackberry, princess tree, *Picia anomala,* and chicory, resorcinols such as 4-hexyl resorcinol, curcuminoids, sugar amines such as N-acetyl glucosamines, and derivatives and mixtures thereof.

Examples of vitamins include, but are not limited to, vitamin A, vitamin B's such as vitamin B3, vitamin B5, and vitamin B12, vitamin C, vitamin K, and different forms of vitamin E like alpha, beta, gamma or delta tocopherols or their mixtures, and derivatives thereof.

Examples of antioxidants include, but are not limited to, water-soluble antioxidants such as sulfhydryl compounds and their derivatives (e.g., sodium metabisulfite and N-acetylcysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

### COMPLEX VISCOSITY TEST METHOD

In one embodiment of the invention, the sunscreen composition has a linear complex viscosity |η*| of about 1500 to about 5000 cP at 100 rad/s. In another embodiment, the sunscreen composition has a linear complex viscosity of about 2000 to about 5000 cP at 100 rad/s. It has been found sunscreen compositions having a linear complex viscosity in this range exhibit excellent spreadibility on the skin, providing a light, watery break during topical application.

Linear complex viscosity (cP) of the sunscreen composition is measured by the following "Complex Viscosity Test Method."

A TA Instruments (New Castle, DE) ARES G2 strain-controlled rheometer equipped with a Peltier temperature control system at 25°C and a parallel plate geometry is used. The linear viscoelastic regime for a sample is determined by performing a strain sweep, where the strain amplitude is increased from 0.1 to 1000% strain at an angular frequency of one radian per second. The end of the linear viscoelastic regime is defined as the strain at which the storage modulus, G', deviates by more than 5% of its average low-strain plateau value. To determine the magnitude of the linear complex viscosity, |η*|, versus frequency, a frequency sweep is performed by varying the angular frequency from 100 to 0.1 radians per second at a strain in the linear viscoelastic regime.

The following examples further illustrate the invention.

### EXAMPLE 1

The following Compositions 1-4 according to the invention and Comparative Compositions A, B, and C were made using the ingredients shown in the following Tables.

**Composition 1 Table**

| **INCI Name** | **Function** | **Weight (%)** |
|---|---|---|
| Disodium EDTA | Chelating agent | 0.1 |
| Glyceryl Stearate | Emulsifier | 0.3 |
| Diisopropyl Adipate | Emollient | 2 |
| Silica | Skin Conditioner | 2 |
| Dicaprylyl Carbonate | Emollient | 1.5 |
| Sodium Hydroxide | pH adjuster | 0.008 |
| Fragrance | Fragrance | 0.3 |
| Aluminum Starch Octenylsuccinate | Skin Conditioner | 0.5 |
| Hydrolyzed Hyaluronic Acid | Humectant | 0.05 |
| Water | Vehicle | 49.08106 |
| Dimethicone;Acrylates/Dimethicone Copolymer | Film Forming Agent | 1.2 |
| Sodium Acryloyldimethyltaurate/VP Crosspolymer | Viscosity Increasing Agent | 0.4 |
| Menthyl Lactate | Refreshing Agent | 0.14 |
| Trideceth-6; Polyurethane-62 | Emulsion Stabilizer | 0.75 |
| Caprylyl Methicone | Skin conditioner occlusive | 3 |
| Blue 1 | Colorant | 0.00044 |
| Alcohol Denat. | Refreshing agent | 3 |
| Tocopheryl Acetate | Antioxidant | 0.1 |
| Homosalate | Sunscreen agent | 9 |
| Oxybenzone | Sunscreen agent | 4.5 |
| Octisalate | Sunscreen agent | 5 |
| Avobenzone | Sunscreen agent | 2.7 |
| Octocrylene | Sunscreen agent | 9 |
| Glycerin | Humectant | 3.5 |
| Chlorphenesin | Preservative | 0.27 |
| Violet 2 | Colorant | 0.0005 |
| Hydroxyacetophenone | Antioxidant | 0.5 |
| Phenoxyethanol | Preservative | 0.6 |
| Pentylene Glycol | Humectant | 0.5 |

Composition 1 was prepared as follows.

Premix 1: Added Hyaluronic acid to 5% Purified Water and mixed using a magnetic stirrer for 1 hour at 35 to 40° C.

Water Phase: Added the remaining Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mixed until fully dissolved.

Oil phase: In a separate container added the following ingredients and heated to target 75-80° C while mixing at 300 rpm: Avobenzone, Homosalate, Octisalate, Octocrylene, Oxybenzone, Caprylyl Methicone, Phenoxyethanol, Diisopropyl Adipate, Caprylyl Carbonate, Glyceryl Stearate, Tocopheryl Acetate, Dimethicone; Acrylates/Dimethicone Copolymer.

At 60°C added Sodium Acryloyldimethyltaurate/VP Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Menthyl Lactate, Ethanol, Violet #2, Blue #1, Pentylene Glycol, Fragrance, Silica, Aluminum Starch Octenylsuccinate and Premix 1. Batch was homogenized for 3 minutes at 4500 rpm. Next measured the pH of the batch, and adjusted to 5.5 with 50% Sodium Hydroxide solution.

**Composition 2 Table**

| **INCI Name** | **Function** | **Weight (%)** |
|---|---|---|
| Water | Vehicle | 62.6717 |
| Trideceth-6; Polyurethane-62 | Emulsion Stabilizer | 0.6 |
| Disodium EDTA | Chelating Agent | 0.1 |
| Glycerin | Humectant | 5 |
| Sodium Acryloyldimethyltaurate/VP Crosspolymer | Viscosity Increasing Agent | 0.6 |
| Hydrolyzed Hyaluronic Acid | Humectant | 0.05 |
| Chlorphenesin | Preservative | 0.27 |
| Hydroxyacetophenone | Antioxidant | 0.5 |
| Homosalate | Sunscreen agent | 5 |
| Octisalate | Sunscreen agent | 4 |
| Octocrylene | Sunscreen agent | 6 |
| Phenoxyethanol | Preservative | 0.3 |
| Dimethicone;Dimethicone Crosspolymer | Skin conditioner | 1 |
| Diisopropyl Adipate | Emollient | 3.5 |
| Caprylyl Methicone | Skin Conditioner occlusive | 2 |
| Dicaprylyl Carbonate | Emollient | 1 |
| Avobenzone | Sunscreen agent | 1.5 |
| Glyceryl Stearate | Emulsifier | 0.3 |
| Tocopheryl Acetate | Antioxidant | 0.1 |
| Dimethicone;Acrylates/Dimethicone Copolymer | Film Former | 1.2 |
| Silica | Skin Conditioner | 1.5 |
| Alcohol Denat. | Refreshing Agent | 2 |
| Blue 1 | Colorant | 0.0003 |
| Fragrance | Fragrance | 0.3 |
| Pentylene Glycol | Humectant | 0.5 |
| Sodium Hydroxide | pH adjuster | 0.008 |

Composition 2 was prepared as follows.

Premix 1: Added Hyaluronic acid to 5% Purified Water and mixed using a magnetic stirrer for 1 hour at 35 to 40° C.

Water Phase: Added the remaining Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mix until fully dissolved.

Oil phase: In a separate container added the following ingredients and heated to target 75-80° C while mixing at 300 rpm: Avobenzone, Homosalate, Octisalate, Octocrylene, Caprylyl Methicone, Phenoxyethanol, Diisopropyl Adipate, Caprylyl Carbonate, Glyceryl Stearate, Tocopheryl Acetate, Dimethicone; Acrylates/Dimethicone Copolymer, Dimethicone; Dimethicone Crosspolymer.

At 60°C added Sodium Acryloyldimethyltaurate/VP Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Blue #1, Pentylene Glycol, Fragrance, Ethanol, Silica and Premix 1. Batch was homogenized for 3 minutes at 4500 rpm. Next measured the pH of the batch, and adjusted to 5.5 with 50% Sodium Hydroxide solution.

**Composition 3 Table**

| **US INCI** | **Function** | **Weight %** |
|---|---|---|
| Water | Vehicle | 49.8697 |
| Hydrolyzed Hyaluronic Acid | Skin Conditioner | 0.05 |
| Disodium EDTA | Chelating Agent | 0.1 |
| Glycerin | Humectant | 3.5 |
| Polyurethane-62; Trideceth-6 | Emulsion stabilizer | 0.7 |
| Chlorphenesin | Preservative | 0.27 |
| Sodium Acryloyldimethyltaurate/VP Crosspolymer | Viscosity Increasing Agent | 0.4 |
| Tocopheryl Acetate | Antioxidant | 0.1 |
| Dimethicone;Acrylates/Dimethicone Copolymer | Film Forming Agent | 1.2 |
| Avobenzone | Sunscreen Agent | 2.7 |
| Homosalate | Sunscreen Agent | 9 |
| Oxybenzone | Sunscreen Agent | 4.5 |
| Octisalate | Sunscreen Agent | 5 |
| Octocrylene | Sunscreen Agent | 9 |
| Caprylyl Methicone | Skin Conditioner Occlusive | 3 |
| Glyceryl Stearate | Emulsifier | 0.3 |
| Phenoxyethanol | Preservative | 0.6 |
| Dicaprylyl Carbonate | Emollient | 1.5 |
| Diisopropyl Adipate | Emollient | 2 |
| Aluminum Starch Octenylsuccinate | Skin Conditioner | 0.5 |
| Silica | Skin Conditioner | 2 |
| Hydroxyacetophenone | Antioxidant | 0.5 |
| Alcohol Denat. | Refreshing Agent | 3 |
| Menthyl Lactate | Refreshing Agent | 0.14 |
| Blue 1 | Colorant | 0.0003 |
| Fragrance | Fragrance | 0.07 |

Composition 3 was prepared as follows.

Premix 1: Added Hyaluronic acid to 5% Purified Water and mixed using a magnetic stirrer for 1 hour at 35 to 40° C.

Water Phase: Added the remaining Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mixed until fully dissolved.

Oil phase: In a separate container added the following ingredients and heated to target 75-80° C while mixing at 300 rpm: Avobenzone, Homosalate, Octisalate, Octocrylene, Oxybenzone, Caprylyl Methicone, Phenoxyethanol, Diisopropyl Adipate, Caprylyl Carbonate, Glyceryl Stearate, Tocopheryl Acetate, Dimethicone; Acrylates/Dimethicone Copolymer.

At 60°C added Sodium Acryloyldimethyltaurate/VP Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Menthyl Lactate, Ethanol, Blue #1, Fragrance, Silica, Aluminum Starch Octenylsuccinate and Premix 1. Batch was homogenized for 3 minutes at 4500 rpm. Next measured the pH of the batch, and adjusted to 5.5 with 50% Sodium Hydroxide solution.

**Composition 4 Table**

| **US INCI** | **Function** | **Percentage** |
|---|---|---|
| Water | Vehicle | 63.399 |
| Trideceth-6; Polyurethane-62 | Emulsion stabilizer | 0.60 |
| Disodium EDTA | Chelating agent | 0.10 |
| Glycerin | Humectant | 5.00 |
| Sodium Acryloyldimethyltaurate/VP Crosspolymer | Viscosity Increasing agent | 0.60 |
| Hydrolyzed Hyaluronic Acid | Skin Conditioner | 0.05 |
| Chlorphenesin | Preservative | 0.27 |
| Hydroxyacetophenone | Antioxidant | 0.50 |
| Homosalate | Sunscreen agent | 5.00 |
| Octisalate | Sunscreen agent | 4.00 |
| Octocrylene | Sunscreen agent | 6.00 |
| Phenoxyethanol | Preservative | 0.30 |
| Dimethicone;Dimethicone Crosspolymer | Skin Conditioner | 1.00 |
| Diisopropyl Adipate | Emollient | 3.50 |
| Caprylyl Methicone | Skin Conditioner Occlusive | 2.00 |
| Dicaprylyl Carbonate | Emollient | 1.00 |
| Avobenzone | Sunscreen Agent | 1.50 |
| Glyceryl Stearate | Emulsifier | 0.30 |
| Tocopheryl Acetate | Antioxidant | 0.10 |
| Dimethicone;Acrylates/Dimethicone Copolymer | Film Former | 1.20 |
| Silica | Skin Conditioner | 1.50 |
| Alcohol Denat. | Refreshing agent | 2.00 |
| Blue 1 | Colorant | 0.0003 |
| Fragrance | Fragrance | 0.07 |
| Sodium Hydroxide | pH adjuster | 0.01 |

Composition 4 was prepared as follows.

Premix 1: Added Hyaluronic acid to 5% Purified Water and mixed using a magnetic stirrer for 1 hour at 35 to 40° C.

Water Phase: Added the remaining Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mix until fully dissolved.

Oil phase: In a separate container added the following ingredients and heated to target 75-80° C while mixing at 300 rpm: Avobenzone, Homosalate, Octisalate, Octocrylene, Caprylyl Methicone, Phenoxyethanol, Diisopropyl Adipate, Caprylyl Carbonate, Glyceryl Stearate, Tocopheryl Acetate, Dimethicone; Acrylates/Dimethicone Copolymer, Dimethicone; Dimethicone Crosspolymer.

At 60°C added Sodium Acryloyldimethyltaurate/VP Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Blue #1, Fragrance, Ethanol, Silica and Premix 1. Batch was homogenized for 3 minutes at 4500 rpm. Next measured the pH of the batch, and adjusted to 5.5 with 50% Sodium Hydroxide solution.

**Comparative Composition A Table**

| US INCI | Function | Weight (%) |
|---|---|---|
| Water | Vehicle | 49.4897 |
| Hydrolyzed Hyaluronic Acid | Skin conditioner | 0.05 |
| Disodium EDTA | Chelating agent | 0.1 |
| Glycerin | Humectant | 3.5 |
| Trideceth-6; Polyurethane-62 | Emulsion stabilizer | 0.85 |
| Chlorphenesin | Preservative | 0.27 |
| Sodium Acryloyldimethyltaurate/VP Crosspolymer | Viscosity increasing agent | 0.5 |
| Tocopheryl Acetate | Skin Conditioner | 0.1 |
| Dimethicone;Acrylates/Dimethicone Copolymer | Film former | 1.2 |
| Avobenzone | Sunscreen agent | 2.7 |
| Homosalate | Sunscreen agent | 9 |
| Oxybenzone | Sunscreen agent | 4.5 |
| Octisalate | Sunscreen agent | 5 |
| Octocrylene | Sunscreen agent | 9 |
| Caprylyl Methicone | Skin conditioner occlusive | 3 |
| Glyceryl Stearate | Emulsifier | 0.3 |
| Phenoxyethanol | Presevative | 0.6 |
| Dicaprylyl Carbonate | Emollient | 1.5 |
| Diisopropyl Adipate | Emollient | 2 |
| Aluminum Starch Octenylsuccinate | Skin Conditioning Agent | 0.5 |
| Silica | Skin Conditioning Agent | 2 |
| Hydroxyacetophenone | Anti-oxidant | 0.5 |
| Alcohol Denat. | Refreshing Agent | 3 |
| Menthyl Lactate | Refreshing Agent | 0.14 |
| Blue 1 | Colorant | 0.0003 |
| Fragrance | Fragrance | 0.2 |

Comparative Composition A was prepared as follows.

Premix 1: Added Hyaluronic acid to 5% Purified Water and mixed using a magnetic stirrer for 1 hour at 35 to 40° C.

Water Phase: Added the remaining Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mix until fully dissolved.

Oil phase: In a separate container added the following ingredients and heated to target 75-80° C while mixing at 300 rpm: Avobenzone, Homosalate, Octisalate, Octocrylene, Oxybenzone, Caprylyl Methicone, Phenoxyethanol, Diisopropyl Adipate, Caprylyl Carbonate, Glyceryl Stearate, Tocopheryl Acetate, Dimethicone; Acrylates/Dimethicone Copolymer.

At 60°C added Sodium Acryloyldimethyltaurate/VP Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Blue #1, Pentylene Glycol, Fragrance, Ethanol, Silica, Aluminum Starch Octenylsuccinate, Menthyl lactate and Premix 1. Batch was homogenized for 3 minutes at 4500 rpm. Next measured the pH of the batch, and adjusted to 5.5 with 50% Sodium Hydroxide solution.

**Comparative Composition B Table**

| **US INCI Name** | **Function** | **Weight (%)** |
|---|---|---|
| Water | Vehicle | 62.27 |
| Trideceth-6; Polyurethane-62 | Emulsion stabilizer | 1.00 |
| Disodium EDTA | Chelating agent | 0.10 |
| Glycerin | Humectant | 5.00 |
| Sodium Acryloyldimethyltaurate/VP Crosspolymer | Viscosity increasing agent | 0.60 |
| Hydrolyzed Hyaluronic Acid | Skin Conditioner | 0.05 |
| Chlorphenesin | Preservative | 0.27 |
| Hydroxyacetophenone | Antioxidant | 0.50 |
| Homosalate | Sunscreen Agent | 5.00 |
| Octisalate | Sunscreen Agent | 4.00 |
| Octocrylene | Sunscreen Agent | 6.00 |
| Phenoxyethanol | Preservative | 0.30 |
| Dimethicone;Dimethicone Crosspolymer | Film Former | 1.00 |
| Diisopropyl Adipate | Emollient | 3.50 |
| Caprylyl Methicone | Skin Conditioner occlusive | 2.00 |
| Dicaprylyl Carbonate | Emollient | 1.00 |
| Avobenzone | Sunscreen Agent | 1.50 |
| Glyceryl Stearate | Emulsifier | 0.30 |
| Tocopheryl Acetate | Skin Conditioner | 0.10 |
| Dimethicone;Acrylates/Dimethicone Copolymer | Skin Conditioner | 1.20 |
| Silica | Skin Conditioner | 1.50 |
| Alcohol Denat. | Refreshing Agent | 2.00 |
| Blue 1 | Colorant | 0.00 |
| Fragrance | Fragrance | 0.30 |
| Pentylene Glycol | Humectant | 0.50 |
| Sodium Hydroxide | pH adjuster | 0.01 |

Comparative Composition B was prepared as follows.

Premix 1: Added Hyaluronic acid to 5% Purified Water and mixed using a magnetic stirrer for 1 hour at 35 to 40° C.

Water Phase: Added the remaining Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mix until fully dissolved.

Oil phase: In a separate container added the following ingredients and heated to target 75-80° C while mixing at 300 rpm: Avobenzone, Homosalate, Octisalate, Octocrylene, Caprylyl Methicone, Phenoxyethanol, Diisopropyl Adipate, Caprylyl Carbonate, Glyceryl Stearate, Tocopheryl Acetate, Dimethicone; Acrylates/Dimethicone Copolymer, Dimethicone; Dimethicone Crosspolymer.

At 60°C added Sodium Acryloyldimethyltaurate/VP Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Blue #1, Pentylene Glycol, Fragrance, Ethanol, Silica and Premix 1. Batch was homogenized for 3 minutes at 4500 rpm. Next measured the pH of the batch, and adjusted to 5.5 with 50% Sodium Hydroxide solution.

**Comparative Composition C Table**

| **US INCI** | **Function** | **Weight (%)** |
|---|---|---|
| Water | Vehicle | 64.96 |
| Trideceth-6; Polyurethane-62 | Emulsion Stabilizer | 0.6 |
| Disodium EDTA | Chelating Agent | 0.1 |
| Glycerin | Humectant | 5 |
| Sodium Acryloyldimethyltaurate/VP Crosspolymer | Viscosity Increasing Agent | 0.5 |
| Chlorphenesin | Preservative | 0.27 |
| Homosalate | Sunscreen Agent | 5 |
| Octisalate | Sunscreen Agent | 4 |
| Octocrylene | Sunscreen Agent | 6 |
| Phenoxyethanol | Preservative | 0.3 |
| Diisopropyl Adipate | Emollient | 3.5 |
| Caprylyl Methicone | Emollient | 2 |
| Dicaprylyl Carbonate | Skin Conditioner | 1 |
| Avobenzone | Sunscreen Agent | 1.5 |
| Dimethicone;Acrylates/Dimethicone Copolymer | Film Former | 1.2 |
| Silica | Skin Conditioner | 1.5 |
| Hydroxyacetophenone | Antioxidant | 0.5 |
| Alcohol Denat. | Refreshing Agent | 2 |
| Fragrance | Fragrance | 0.07 |

Comparative Composition C was prepared as follows.

Water Phase: Added the remaining Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mix until fully dissolved.

Oil phase: In a separate container added the following ingredients and heated to target 75-80° C while mixing at 300 rpm: Avobenzone, Homosalate, Octisalate, Octocrylene, Caprylyl Methicone, Phenoxyethanol, Diisopropyl Adipate, Caprylyl Carbonate, Dimethicone; Acrylates/Dimethicone Copolymer.

At 60°C added Sodium Acryloyldimethyltaurate/VP Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Fragrance, Ethanol and Silica. Batch was homogenized for 3 minutes at 4500 rpm. Next measured the pH of the batch, and adjusted to 5.5 with 50% Sodium Hydroxide solution.

Comparative Composition C did not possess the expected aesthetics to be included in the testing. Visual thickness was above the target.

### EXAMPLE 2

The complex viscosity of Compositions 1-4 and Comparative Compositions A and B were tested using the Complex Viscosity Test Method set forth above.

In addition, each of these compositions was evaluated for skin feel rub-out sensory characteristics using the following method. With the support of a cosmetic spatula to the back of the non-dominant hand, 0.10 grams (about the size of a pea) of the composition was rubbed in with fingers of the dominant hand for five rotations and applied for five minutes before sensory evaluation was taken. The overall ease of application and rate of dry out were determined.

The results are shown in Table 2.

**Table 2**

| **Test Composition** | **Linear complex viscosity \|η*\| at 100 rad/s (cP)** | **Rub-out sensory characteristics** |
|---|---|---|
| | | |
| Composition 1 | | Easy to apply, watery break |
| Composition 2 | 2840 | Easy to apply, watery break |
| Composition 3 | 3030 | Easy to apply, watery break |
| Composition 4 | 2370 | Easy to apply, watery break |
| Comparative Composition A | 5790 | Difficult to apply, thick, no watery break |
| Comparative Composition B | 1980 | Too runny to apply easily |

Only Compositions 1-4 containing a combination of about 0.55 to about 0.75 weight percent of a hydrophobically modified polyurethane; about 0.4 to about 0.6 weight percent of a taurate copolymer; and at least about 0.1 weight percent of glyceryl stearate had linear complex viscosities of 2000-5000.

Consistent with that, Compositions 1-4 required little effort to apply and provided a watery break during rubbing, delivering a fresh feeling upon application and a quick dry out, leaving a matte and refreshing finish. In contrast, Comparative Composition A required more effort to apply and also lacked a watery break due to its thickness, while Comparative Composition B was too runny to be applied easily and uniformly, and also took too long to apply and dry out.

### REFERENCE EXAMPLE 3

The following Compositions 5-8 and Comparative Composition D was made using the ingredients shown in the following Tables.

**Composition 5 Table**

| **INCI Name** | **Function** | **Weight (%)** |
|---|---|---|
| Water | Vehicle | 61.29 |
| Disodium EDTA | Chelating agent | 0.1 |
| Glycerin | Humectant | 3 |
| Polyurethane-62; Trideceth-6 | Viscosity increasing agent | 0.8 |
| Hydroxyacetophenone | Antioxidant | 0.5 |
| Styrene/Acrylates Copolymer | Opacifying Agent | 2.5 |
| Acrylates/C10-30 Alkyl Acrylate Crosspolymer | viscosity increasing agent | 0.2 |
| Butyl Methoxydibenzoylmethane | Sunscreen agent | 2.25 |
| Homosalate | Sunscreen agent | 8 |
| Bis-Ethylhexyloxyphenol Methoxyphenyl Triazine | Sunscreen agent | 2 |
| Glyceryl Stearate | Emulsifier | 0.3 |
| Octocrylene | Sunscreen agent | 6 |
| Caprylyl Methicone | Emollient | 2 |
| Phenoxyethanol | Preservative | 0.7 |
| Dimethicone; Acrylates/Dimethicone Copolymer | Film Forming Agent | 1.2 |
| Dicaprylyl Carbonate | Emollient | 1.5 |
| Diisopropyl Adipate | Emollient | 2 |
| Tocopheryl Acetate | Antioxidant | 0.5 |
| Sodium Hydroxide | pH adjuster | 0.11 |
| Silica | Skin Conditioner | 2 |
| Pentylene Glycol | Humectant | 0.5 |
| Fragrance | Fragrance | 0.35 |
| Menthyl Lactate | Fragrance | 0.2 |
| Alcohol | Solvent | 2 |

Composition 5 was prepared as follows.

Water Phase: Added Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Hydroxyacetophenone under stirring and mixed until fully dissolved.

Oil phase: In a separate container added the following ingredients and heated to target 75-80° C while mixing at 300 rpm: Butyl Methoxydibenzoylmethane,

Homosalate, Bis-Ethylhexyloxyphenol Methoxyphenyl Triazine, Caprylyl Methicone, Octocrylene, Phenoxyethanol, Diisopropyl Adipate, Caprylyl Carbonate, Glyceryl Stearate, Tocopheryl Acetate, Dimethicone; Acrylates/Dimethicone Copolymer and Styrene/Acrylates Copolymer.

At 60°C added Acrylates/C10-30 Alkyl Acrylate Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Menthyl Lactate, Ethanol, Pentylene Glycol, Fragrance and Silica. Batch was homogenized for 3 minutes at 4500 rpm. Next measured the pH of the batch, and adjusted to 5.5 with 50% Sodium Hydroxide solution.

**Composition 6 Table**

| **INCI Name** | **Function** | **Weight (%)** |
|---|---|---|
| Water | Vehicle | 54.49 |
| Disodium EDTA | Chelating agent | 0.10 |
| Glycerin | Humectant | 3.00 |
| Polyurethane-62; Trideceth-6 | Viscosity increasing agent | 0.8 |
| Hydroxyacetophenone | Antioxidant | 0.5 |
| Styrene/Acrylates Copolymer | Opacifying Agent | 2.5 |
| Acrylates/C10-30 Alkyl Acrylate Crosspolymer | Viscosity increasing agent | 0.2 |
| Butyl Methoxydibenzoylmethane | Sunscreen agent | 3.7 |
| Homosalate | Sunscreen agent | 9.0 |
| Bis-Ethylhexyloxyphenol Methoxyphenyl Triazine | Sunscreen agent | 3 |
| Potassium Cetyl Phosphate; Hydrogenated Palm Glycerides | Emulsifier | 0.35 |
| Glyceryl Stearate | Emulsifier | 0.30 |
| Octocrylene | Sunscreen agent | 9.0 |
| Caprylyl Methicone | Emollient | 2.0 |
| Phenoxyethanol | Preservative | 0.70 |
| Dimethicone; Acrylates/Dimethicone Copolymer | Film Forming Agent | 1.20 |
| Dicaprylyl Carbonate | Emollient | 1.50 |
| Diisopropyl Adipate | Emollient | 2.0 |
| Tocopheryl Acetate | Antioxidant | 0.5 |
| Sodium Hydroxide | pH adjuster | 0.11 |
| Silica | Skin Conditioner | 2.00 |
| Pentylene Glycol | Humectant | 0.5 |
| Fragrance | Fragrance | 0.35 |
| Menthyl Lactate | Fragrance | 0.20 |
| Alcohol | Solvent | 2.00 |

Composition 6 was prepared as follows.

Water Phase: Added Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Hydroxyacetophenone under stirring and mix until fully dissolved.

Oil phase: In a separate container added the following ingredients and heated to target 75-80° C while mixing at 300 rpm: Butyl Methoxydibenzoylmethane,
Homosalate, Bis-Ethylhexyloxyphenol Methoxyphenyl Triazine, Caprylyl Methicone, Octocrylene, Phenoxyethanol, Diisopropyl Adipate, Caprylyl Carbonate, Glyceryl Stearate, Tocopheryl Acetate, Dimethicone; Acrylates/Dimethicone Copolymer, Styrene/Acrylates Copolymer and Potassium Cetyl Phosphate; Hydrogenated Palm Glycerides.

At 60°C added Acrylates/C10-30 Alkyl Acrylate Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Pentylene Glycol, Fragrance, Ethanol and Silica. Batch was homogenized for 3 minutes at 4500 rpm. Next measured the pH of the batch, and adjusted to 5.5 with 50% Sodium Hydroxide solution.

**Composition 7 Table**

| **INCI Name** | **Function** | **Weight (%)** |
|---|---|---|
| Water | Vehicle | 61.29 |
| Disodium EDTA | Chelating agent | 0.1 |
| Glycerin | Humectant | 3 |
| Polyurethane-62; Trideceth-6 | Viscosity increasing agent | 0.8 |
| Hydroxyacetophenone | Antioxidant | 0.5 |
| Styrene/Acrylates Copolymer | Opacifying Agent | 2.5 |
| Acrylates/C10-30 Alkyl Acrylate Crosspolymer | Viscosity increasing agent | 0.2 |
| Butyl Methoxydibenzoylmethane | Sunscreen agent | 2.25 |
| Homosalate | Sunscreen agent | 8 |
| Bis-Ethylhexyloxyphenol Methoxyphenyl Triazine | Sunscreen agent | 2 |
| Glyceryl Stearate | Emulsifier | 0.3 |
| Octocrylene | Sunscreen agent | 6 |
| Caprylyl Methicone | Emollient | 2 |
| Phenoxyethanol | Emollient | 0.7 |
| Dimethicone; Acrylates/Dimethicone Copolymer | Emollient | 1.2 |
| Dicaprylyl Carbonate | Film Forming Agent | 1.5 |
| Diisopropyl Adipate | Emollient | 2 |
| Tocopheryl Acetate | Antioxidant | 0.5 |
| Sodium Hydroxide | pH adjuster | 0.11 |
| Silica | Skin Conditioner | 2 |
| Pentylene Glycol | Humectant | 0.5 |
| Fragrance | Fragrance | 0.35 |
| Menthyl Lactate | Fragrance | 0.2 |
| Alcohol | Solvent | 2 |

Composition 7 was prepared as follows.

Water Phase: Added Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Hydroxyacetophenone under stirring and mixed until fully dissolved.

Oil phase: In a separate container added the following ingredients and heated to target 75-80° C while mixing at 300 rpm: Butyl Methoxydibenzoylmethane,

Homosalate, Bis-Ethylhexyloxyphenol Methoxyphenyl Triazine, Caprylyl Methicone, Octocrylene, Phenoxyethanol, Diisopropyl Adipate, Caprylyl Carbonate, Glyceryl Stearate, Tocopheryl Acetate, Dimethicone; Acrylates/Dimethicone Copolymer and Styrene/Acrylates Copolymer.

At 60°C added Acrylates/C10-30 Alkyl Acrylate Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Menthyl Lactate, Ethanol, Fragrance and Silica. Batch was homogenized for 3 minutes at 4500 rpm. Next measured the pH of the batch, and adjusted to 5.5 with 50% Sodium Hydroxide solution.

**Composition 8 Table**

| **INCI Name** | **Function** | **Weight (%)** |
|---|---|---|
| Water | Vehicle | 55.92 |
| Disodium EDTA | Chelating agent | 0.1 |
| Glycerin | Humectant | 3 |
| Chlorphenesin | Preservative | 0.2 |
| Trideceth-6; Polyurethane-62 | Viscosity increasing agent | 0.7 |
| Acrylates/C10-30 Alkyl Acrylate Crosspolymer | Viscosity increasing agent | 0.18 |
| Butyl Methoxydibenzoylmethane | Sunscreen agent | 2.5 |
| Homosalate | Sunscreen agent | 9 |
| Ethylhexyl Salicylate | Sunscreen agent | 4 |
| Bis-Ethylhexyloxyphenol Methoxyphenyl Triazine | Sunscreen agent | 2.5 |
| Glyceryl Stearate | Emulsifier | 0.3 |
| Octocrylene | Sunscreen agent | 9 |
| Dimethicone; Acrylates/Dimethicone Copolymer | Emollient | 1.2 |
| Caprylyl Methicone | Emollient | 1 |
| Phenoxyethanol | Preservative | 0.7 |
| Diisopropyl Adipate | Emollient | 2 |
| Dicaprylyl Carbonate | Emollient | 1.5 |
| Polymethylsilsesquioxane | Emollient | 2 |
| Ethylhexylglycerin | Skin Conditioner | 1 |
| Fragrance | Fragrance | 0.2 |
| Alcohol | Solvent | 3 |

Composition 8 was prepared as follows.

Water Phase: Added Purified Water, Disodium EDTA and Glycerin to the main vessel and mixed at 300 rpm until completely dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mix until fully dissolved.

Oil phase: In a separate container added the following ingredients and heated to target 75-80° C while mixing at 300 rpm: Butyl Methoxydibenzoylmethane, Homosalate, Bis-Ethylhexyloxyphenol Methoxyphenyl Triazine, Ethylhexyl Salicylate, Caprylyl Methicone, Octocrylene, Phenoxyethanol, Diisopropyl Adipate, Caprylyl Carbonate, Glyceryl Stearate, Tocopheryl Acetate, Dimethicone; Acrylates/Dimethicone Copolymer, Ethylhexylglycerin.

At 60°C added Acrylates/C10-30 Alkyl Acrylate Crosspolymer.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Fragrance, Ethanol and Polymethylsilsesquioxane. Batch was homogenized for 3 minutes at 4500 rpm. Next measured the pH of the batch, and adjusted to 5.5 with 50% Sodium Hydroxide solution.

**Comparative Composition D Table**

| **INCI Name** | **Function** | **Weight (%)** |
|---|---|---|
| Water | Vehicle | 52.9 |
| Disodium EDTA | Chelating agent | 0.1 |
| Glycerin | Humectant | 3 |
| Chlorphenesin | Preservative | 0.2 |
| Phenylbenzimidazole Sulfonic Acid | Sunscreen agent | 2 |
| Sodium Hydroxide | pH adjuster | 0.6 |
| Trideceth-6; Polyurethane-62 | Viscosity increasing agent | 0.7 |
| Sodium Polyacrylate | Viscosity increasing agent | 0.3 |
| Butyl Methoxydibenzoylmethane | Sunscreen agent | 2.5 |
| Homosalate | Sunscreen agent | 9 |
| Ethylhexyl Salicylate | Sunscreen agent | 4 |
| Bis-Ethylhexyloxyphenol Methoxyphenyl Triazine | Sunscreen agent | 2.5 |
| Glyceryl Stearate | Emulsifier | 0.3 |
| Octocrylene | Sunscreen agent | 7 |
| Dimethicone; Acrylates/Dimethicone Copolymer | Emollient | 1.2 |
| Caprylyl Methicone | Emollient | 3 |
| Phenoxyethanol | Preservative | 0.7 |
| Diisopropyl Adipate | Emollient | 2 |
| Tocopheryl Acetate | Emollient | 0.1 |
| Dicaprylyl Carbonate | Emollient | 1.5 |
| Polymethylsilsesquioxane | Emollient | 2 |
| Ethylhexylglycerin | Skin Conditioner | 1 |
| Fragrance | Fragrance | 0.2 |
| Menthyl Lactate | Fragrance | 0.2 |
| Alcohol | Solvent | 3 |

Comparative Composition D was prepared as follows.

Water Phase: Added Purified Water, Disodium EDTA, Glycerin and sodium Hydroxide to the main vessel and mixed at 300 rpm until completely dissolved. Added Phenylbenzimidazole Sulfonic Acid under mixing until fully dissolved. Started heating up to 50°C and slowly sprinkled Trideceth-6; Polyurethane-62 under mixing at 300 rpm. Once the Polyurethane-62 was fully hydrated, increased temperature to 75-80°C. Maintained temperature between 75-80°C. Added Chlorphenesin and Hydroxyacetophenone under stirring and mix until fully dissolved.

Oil phase: In a separate container added the following ingredients and heated to target 75-80° C while mixing at 300 rpm: Butyl Methoxydibenzoylmethane, Homosalate, Bis-Ethylhexyloxyphenol Methoxyphenyl Triazine, Ethylhexyl Salicylate, Caprylyl Methicone, Octocrylene, Phenoxyethanol, Diisopropyl Adipate, Caprylyl Carbonate, Glyceryl Stearate, Tocopheryl Acetate, Dimethicone; Acrylates/Dimethicone Copolymer and Ethylhexylglycerin.

At 60°C added Sodium Polyacrylate.

Emulsification: when water phase and oil phase were between 75°C and 80°C slowly added oil phase to water phase under vigorous agitation and mixing at 1000 rpm. Homogenized for 4 minutes at 4500 rpm. Began to cool batch below 40°C mixing at 800 rpm.

Post Adds: once the batch was below 40°C, added Fragrance, Ethanol, Menthyl lactate and Polymethylsilsesquioxane. Batch was homogenized for 3 minutes at 4500 rpm. Next measured the pH of the batch, and adjusted to 7.0 with 50% Sodium Hydroxide solution.

### REFERENCE EXAMPLE 4

The complex viscosities of Compositions 5-8 and Comparative Composition D were tested using the Complex Viscosity Test Method set forth above.

In addition, each of these compositions was evaluated for skin feel rub-out sensory characteristics using the method set forth in Example 2.

The results are shown in Table 3.

**Table 3**

| **Test Composition** | **Linear complex viscosity \|η*\| at 100 rad/s (cP)** | **Rub-out sensory characteristics** |
|---|---|---|
| | | |
| Composition 5 | 1840 | Easy to apply, watery break |
| Composition 6 | 3935 | Easy to apply, watery break |
| Composition 7 | 2364 | Easy to apply, watery break |
| Composition 8 | 3281 | Easy to apply, watery break |
| Comparative Composition D | Formula separated, unstable, unable to measure | Unstable, phase separation |

Only Compositions 5-8 containing a combination of about 0.55 to about 1 weight percent of a hydrophobically modified polyurethane; about 0.1 to about 0.3 weight percent of an acrylates crosspolymer; and at least about 0.1 weight percent of glyceryl stearate had linear complex viscosities of 1500-5000.

Consistent with that, Compositions 5-8 required little effort to apply and provided a watery break during rubbing, delivering a fresh feeling upon application and a quick dry out, leaving a matte and refreshing finish. In contrast, Comparative Composition D phase separated; it was unstable, and its complex viscosity was unable to be measured.

## Claims

1. A sunscreen composition comprising:
(a) at least 10 weight percent of one or more UV filters;
(b) 0.55 to 0.75 weight percent of a hydrophobically modified polyurethane;
(c) 0.1 to 0.6 weight percent of a viscosity increasing polymer selected from the group consisting of taurate copolymers and acrylate crosspolymers; and
(d) at least 0.1 weight percent of glyceryl stearate;
wherein said viscosity increasing polymer is a taurate copolymer.

2. The composition of claim 1 comprising at least 0.1 weight percent avobenzone.

3. The composition of claim 2 further comprising octocrylene and homosalate.

4. The composition of claim 1 further comprising hyaluronic acid.

5. The composition of claim 1 further comprising silica.

6. The composition of claim 1 further comprising a humectant selected from the group consisting of glycerin, pentylene glycol, and mixtures thereof.

7. The sunscreen composition of claim 1, comprising:
(a) at least 10 weight percent of a combination of UV filters comprising avobenzone, octocrylene, homosalate, and octisalate;
(c) 0.4 to 0.6 weight percent of the taurate copolymer; and
(e) silica;
(f) a humectant selected from the group consisting of glycerin, propylene glycol, and mixtures thereof; and
(g) ethanol.

## Patentansprüche

1. Sonnenschutzzusammensetzung umfassend:
(a) wenigstens 10 Gewichtsprozent an einem oder mehreren UV-Filtern;
(b) 0,55 bis 0,75 Gewichtsprozent an einem hydrophob modifizierten Polyurethan;
(c) 0,1 bis 0,6 Gewichtsprozent an einem viskositätserhöhenden Polymer ausgewählt aus der Gruppe bestehend aus Taurat-Copolymeren und Acrylat-Kreuzpolymeren; und
(d) wenigstens 0,1 Gewichtsprozent Glycerylstearat; wobei das viskositätserhöhende Polymer ein Taurat-Copolymer ist.

2. Zusammensetzung gemäß Anspruch 1, umfassend wenigstens 0,1 Gewichtsprozent Avobenzon.

3. Zusammensetzung gemäß Anspruch 2, ferner umfassend Octocrylen und Homosalat.

4. Zusammensetzung gemäß Anspruch 1, ferner umfassend Hyaluronsäure.

5. Zusammensetzung gemäß Anspruch 1, ferner umfassend Siliciumdioxid.

6. Zusammensetzung gemäß Anspruch 1, ferner umfassend ein Feuchthaltemittel ausgewählt aus der Gruppe bestehend aus Glycerin, Pentylenglycol und Gemischen davon.

7. Sonnenschutzzusammensetzung gemäß Anspruch 1, umfassend:
(a) wenigstens 10 Gewichtsprozent an einer Kombination von UV-Filtern umfassend Avobenzon, Octocrylen, Homosalat und Octisalat;
(c) 0,4 bis 0,6 Gewichtsprozent an dem Taurat-Copolymer; und
(e) Siliciumdioxid;
(f) ein Feuchthaltemittel ausgewählt aus der Gruppe bestehend aus Glycerin, Propylenglycol und Gemischen davon; und
(g) Ethanol.

## Revendications

1. Composition d'écran solaire comprenant :
(a) au moins 10 pour cent en poids d'un ou plusieurs filtres UV ;
(b) 0,55 à 0,75 pour cent en poids d'un polyuréthane modifié hydrophobiquement ;
(c) 0,1 à 0,6 pour cent en poids d'un polymère augmentant la viscosité sélectionné dans le groupe constitué par des copolymères de taurate et des polymères croisés d'acrylate ; et
(d) au moins 0,1 pour cent en poids de stéarate de glycéryle ; dans laquelle ledit polymère augmentant la viscosité est un copolymère de taurate.

2. Composition selon la revendication 1 comprenant au moins 0,1 pour cent en poids d'avobenzone.

3. Composition selon la revendication 2 comprenant en outre de l'octocrylène et de l'homosalate.

4. Composition selon la revendication 1 comprenant en outre de l'acide hyaluronique.

5. Composition selon la revendication 1 comprenant en outre de la silice.

6. Composition selon la revendication 1 comprenant en outre un humectant sélectionné dans le groupe constitué par la glycérine, le pentylène glycol et des mélanges correspondants.

7. Composition d'écran solaire selon la revendication 1, comprenant :
(a) au moins 10 pour cent en poids d'une combinaison de filtres UV comprenant de l'avobenzone, de l'octocrylène, de l'homosalate et de l'octisalate ;
(c) 0,4 à 0,6 pour cent en poids du copolymère de taurate ; et
(e) de la silice ;
(f) un humectant sélectionné dans le groupe constitué par la glycérine, le propylène glycol et des mélanges correspondants ; et
(g) de l'éthanol.
